# EUROPEAN PATENT SPECIFICATION

(11) **EP 0 704 243 B1**
(45) Date of publication and mention of the grant of the patent: **09.08.2000**
(21) Application number: 95114275.1
(22) Date of filing: 11.09.1995
(51) Int. Cl.: B01L 9/06, B65D 25/10

(54) **Holder assembly for reaction tubes**
Trägervorrichtung für Teströhrchen
Dispositif de support pour tubes de réaction

(30) Priority: 30.09.1994 US 316299
(43) Date of publication of application: 03.04.1996
(73) Proprietor: THE PERKIN-ELMER CORPORATION, Norwalk Connecticut 06856-0181 (US)
(72) Inventor: Griffin, Reginald, Wrentham, MA 02093 (US)
(74) Representative: Grünecker, Kinkeldey, Stockmair & Schwanhäusser Anwaltssozietät

(56) References cited:
- EP-A- 0 488 769
- DE-A- 2 618 522
- US-A- 3 643 812
- US-A- 4 207 289
- US-A- 4 284 603

## Description

### FIELD OF INVENTION

This invention generally relates to a holder assembly for holding reaction tubes, preferably utilized in an instrument for automated thermal cyclers for performing polymerase chain reactions (PCR).

### BACKGROUND OF THE INVENTION

Automated thermal cyclers for performing PCR simultaneously on a number of samples are disclosed in U.S. Pat. No. 5,038,852. Briefly, PCR is an enzymatic process by which a small amount of specific DNA sequences can be greatly amplified in a relatively short period of time. The method utilizes two oligonucleotide primers that hybridize to opposite strands and flank the region of interest in the target DNA. A repetitive series of thermal cycles involving template denaturation, primer annealing, and the extension of the annealed primers by DNA polymerase results in the exponential accumulation of a specific DNA fragment whose termini are defined by the **5'** ends of the primers.

A reaction mixture made up of the target DNA to be amplified, oligonucleotide primers, buffers, nucleotide triphosphates, and preferably a thermostable enzyme such as Taq polymerase, are combined and placed in reaction tubes. The reaction mixture contained in the tubes is then subjected to a number of thermal transition and soak periods known as PCR protocols in a thermal cycler to generate the amplified target DNA.

An array of reaction tubes is typically made up of up to either twenty four or forty eight or ninety six tubes arranged in a 8 x 3 array or a 6 x 8 array or an 8 x 12 array in a tray. The array of tubes is placed in a metal thermal cycler block so that the lower portion of each tube is in intimate thermal contact with the block.

The temperature of the block is then varied in accordance with the predetermined temperature/time profile of the PCR protocol for a predetermined number of cycles.

Holder assemblies for reaction tubes are preferably compatible with microtiter plate format lab equipment while maintaining sufficient individual tube freedom of movement to compensate for differences in the various rates of thermal expansion of the various components.

### SUMMARY OF THE INVENTION

Briefly, this invention contemplates the provision of a new and improved plastic holder assembly for loosely holding a plurality of microtiter sample tubes, which includes a tray having a plurality of holes for receiving the tubes. The tray has opposite vertical end walls, each of the end walls having two spaced vertically extending slots and a horizontally extending recess therebetween. A retainer is provided which releasably nests in the tray. The retainer has a corresponding plurality of holes, and has opposite vertical end walls corresponding to the end walls of the tray. A U-shaped handle extends horizontally outwardly from each of the opposite retainer end walls. Each of the handles have two legs which slide into the tray slots respectively when the retainer is nested in the tray. A tab projects horizontally outwardly from each of the retainer end walls between the legs, which snap into the tray end wall recesses respectively when the retainer is nested in the tray. The retainer has an elongated slot parallel and directly adjacent each of the end walls, whereby inwardly directed finger pressure on the U-shaped handles inwardly flexes the opposite ends of the retainer to release the tabs from the tray recesses respectively to facilitate removal of the retainer from the tray.

According to one aspect of the invention, a plastic base is provided, which has a plurality of wells in a rectangular array, compatible with the holes in the tray and retainer. The wells are dimensioned to snugly accept the lower sections of the tubes. The base is assembled with the tray and retainer and sample tubes to form a microtiter plate assembly having a foot print of a industry standard microtiter plate assembly.

In one form of the invention the tray and retainer have beveled mating corners, thereby to align the retainer with respect to the tray repeatedly in the same orientation.

According to another aspect of the invention, the assembly is fabricated from molded reinforced polyester thermoplastic with the wall sections having a thickness of the order of about 1.27 mm.

These, and other advantages and features of the invention, will become more apparent from a detailed reading of the following description when taken in conjunction with the appended claims.

### BRIEF DESCRIPTION OF THE DRAWINGS

Fig. **1** is an exploded perspective view of a microtiter plate assembly, including the plastic holder assembly according to the invention;
Fig. **2** is a vertical sectional view of a sample tube;
Fig. **3** is a top plan view of the retainer;
Fig. **4** is a sectional view taken through the retainer along section line 4-4 in Fig. **3**;
Fig. **5** is a top plan view of the tray;
Fig. **6** is a sectional view taken through the tray along section line 6-6 in Fig. **5**;
Fig. **7** is a sectional view taken through the tray along section line 7-7 in Fig. **5**;
Fig. **8** is a top plan view of the base;
Fig. **9** is sectional view taken along the line 9-9 in Fig. **8**; and
Fig. **10** is a sectional view taken along the line 10-10 in Fig. **8**.

### DETAILED DESCRIPTION OF THE PRESENTLY PREFERRED EMBODIMENT OF THE INVENTION

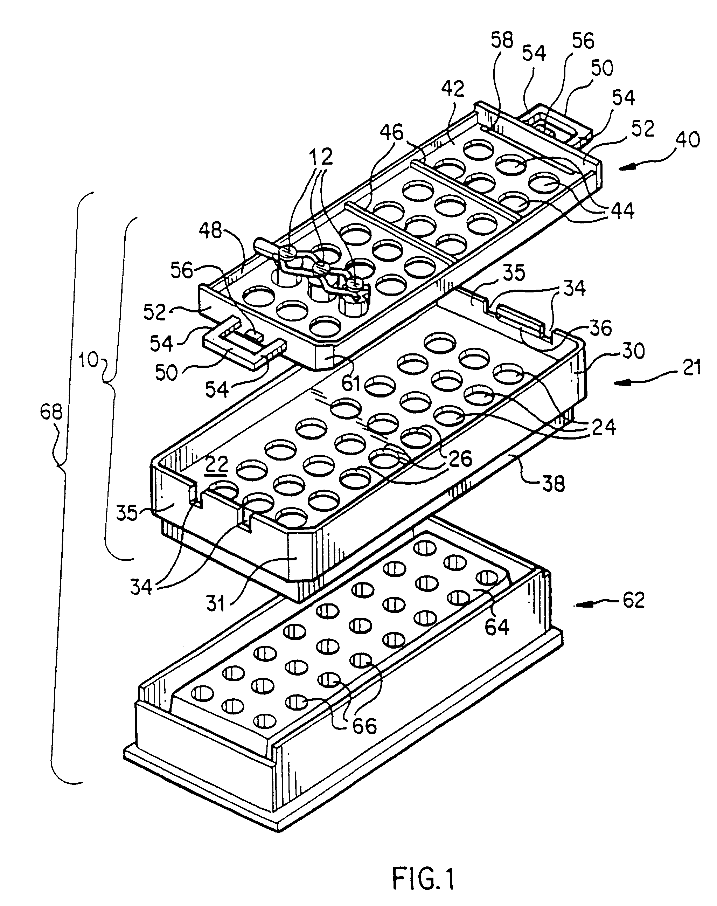

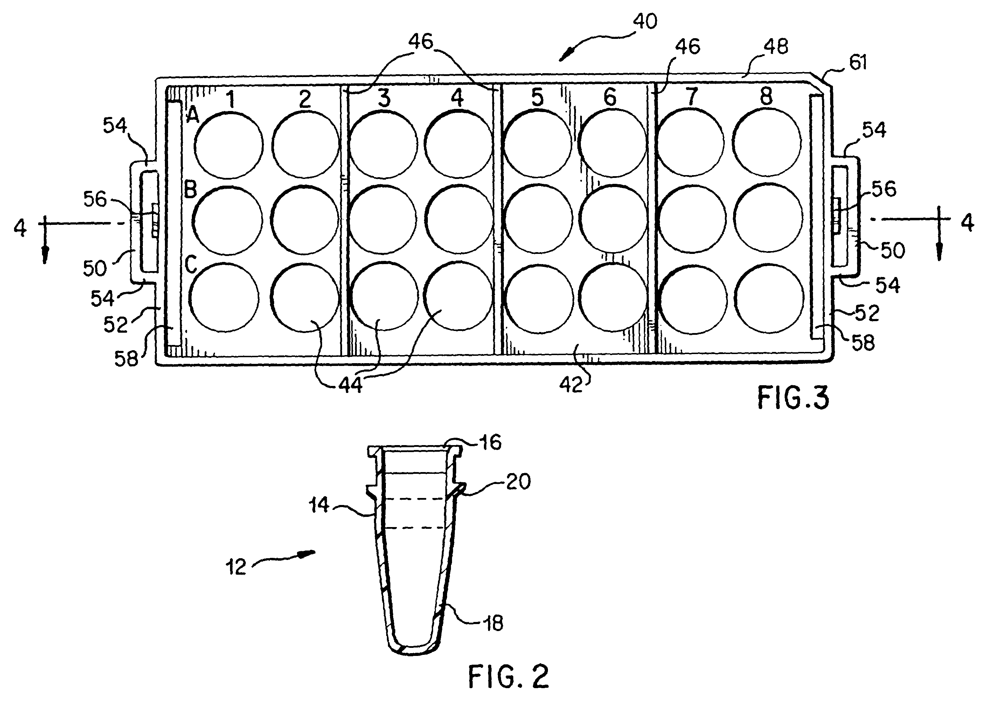

Fig. **1** is an exploded perspective view of a presently preferred embodiment of the invention. A two piece plastic holder assembly, indicated at **10**, loosely holds a plurality of microtiter sample tubes indicated at **12**, Fig. **2**. Each tube has a cylindrical shaped upper section **14** open at its top end **16** and a closed, tapered lower section **18** extending downwardly therefrom. Each tube is of circular cross-section and has a circumferential shoulder **20** extending outwardly from the upper section **14** at a position on the upper section spaced from the open end **16** thereof.

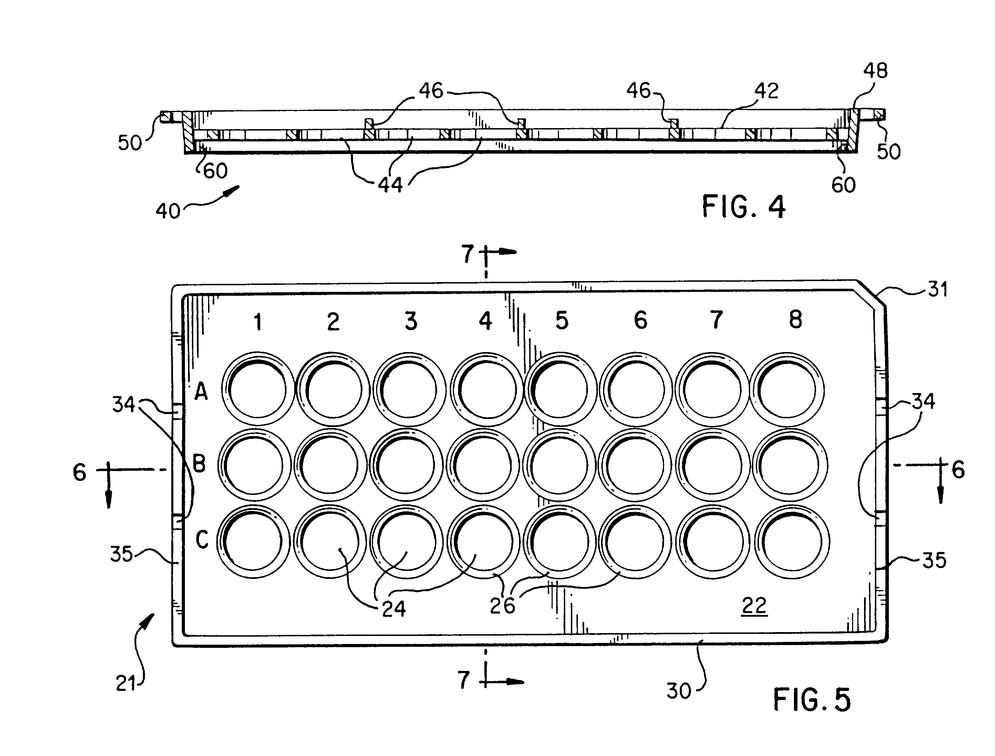

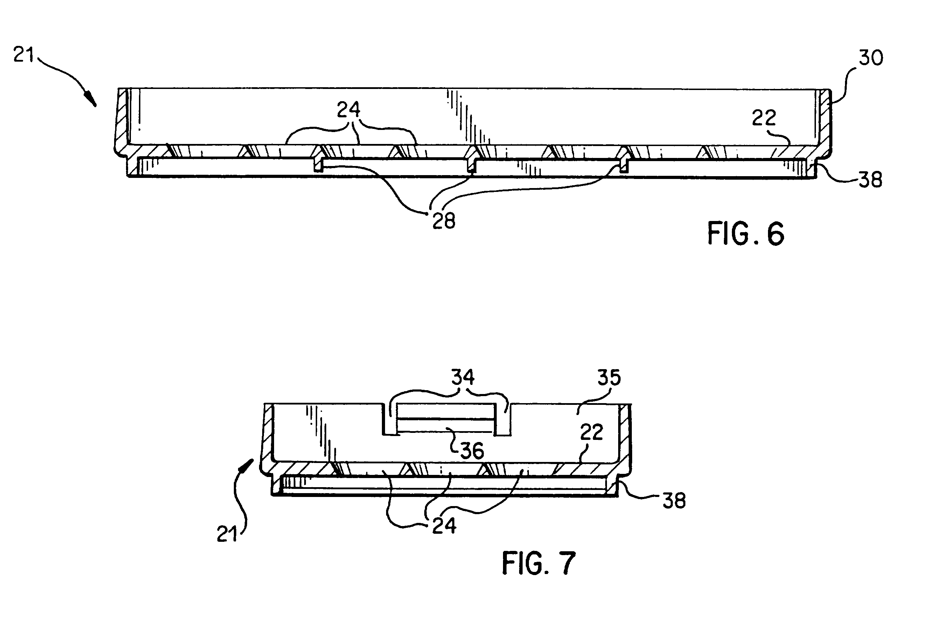

A one-piece tray **21**, as seen in Figs. **1**, **5**, **6** and **7**, comprises a flat, horizontal, rectangular tray plate section **22**, which contains a first plurality of holes **24** in an array compatible with industrial standard microtiter plate format. Figs. **1** and **5** show an array of **24** holes. However, in some installations it may be desirable to have other numbers of holes such as, for example, forty eight or ninety six. The diameters of the holes are larger than the outside diameter of the upper section **14** of the tubes **12**, Fig. **2**, by about 0.7 mm., but are smaller than the outside diameter of the shoulder **20**. The holes are counter-sunk as indicated at **26** in Figs. **1** and **5**. The tray plate **22** has a plurality of support ribs **28**, as best seen in Fig. **6**, between the rows of holes. Three ribs are shown in Fig. **6**.

The tray **21** further includes a vertical tray sidewall section **30** around the plate section **22** extending upwardly to a height greater than the height of a tube **12**, Fig. **1**, resting in one of the holes **24**. Two spaced vertically extending tray sidewall slots **34**, Figs. **1**, **5** and **7** are disposed in each of two opposite ends **35** of the tray sidewall section **30**. An elongated horizontal tray sidewall recess **36** is disposed between each of the two-spaced tray sidewall slots for a purpose two be discussed more fully hereinafter.

The tray **21** also includes a second vertical tray sidewall section **38**, Figs. **1**, **6** and **7**, around the plate section **22** extending downwardly approximately to the bottom of the upper section **14** of a tube **12** resting in one of the holes **24**.

A one-piece rectangular retainer **40**, as seen in Figs. **1**, **3** and **4** is releasably nested in the tray **21** over the sample tubes **32** resting in the tray. This retainer includes a flat, horizontal, rectangular plate section **42**, which contains a second plurality of holes **44** in a rectangular array compatible with the first plurality of holes **24**. That is, the holes **44** are in vertical alignment with the holes **24** when the retainer **40** is nested in the tray **21**. Twenty-four holes are shown, for example. As indicated hereinbefore, this array of holes is compatible with industrial standard microtiter plate format. Holes **44** are larger in diameter than the outside diameter of the upper portion **14** of the tube **12** by about 0.7 mm., but smaller than the outside diameter of the shoulder **20**. This retainer plate section **42** has a plurality of support ribs **46** extending along the upper side of the retainer plate section between the rows of holes.

The retainer **40** has a first vertical retainer sidewall section **48** extending around the plate section **42** and which extends upwardly. A U-shaped handle **50**, figs. **1**, **3**, and **4**, extends horizontally outwardly from each of two opposite ends **52** of the retainer sidewall section **48** corresponding to the two opposite ends **35** of the tray sidewall section **30**. Each of the U-shaped handles **50** has two spaced legs **54**, which slide into the tray slots **34** respectively, when the retainer **40** is nested in the tray **21**. Tabs **56** project horizontally outwardly from the sidewall sections **52** respectively between each of the two legs **54**. These tabs **56** snap into the sidewall recess **36** in the sidewall sections **35** of the tray **21** respectively when the retainer **40** is nested in the tray **21**. The retainer plate section **42** has elongated slots **58**, Figs. **1** and **3**, parallel to and directly adjacent the opposite ends **52** of the retainer sidewall section respectively, whereby inwardly directed finger pressure on the U-shaped handles **50** inwardly flex the opposite ends **52** of the retainer sidewall sections to release the tabs **56** from the tray sidewall recesses **36** respectively, thereby to facilitate removal of the retainer **40** from the tray **21**.

In addition, the retainer **40** has a second vertical retainer sidewall section **60**, Fig. 4, extending around the retainer plate section **42** and extending downwardly from the retainer plate section.

The tray **21** of Figs. **5-7**, with up to twenty-four sample tubes **12** placed therein and with the retainer **40** snapped into position, forms a single unit **10**, which can be placed in a PCR instrument for processing. When the retainer **40** is nested in the tray **21**, the retainer plate section **42** lies slightly above the shoulder **20**, Fig. **2**, of a tube resting in the tray and the first tray sidewall section **30** is about as high as the retainer sidewall section **48**, whereby tubes resting in the tray are retained loosely both vertically and laterally.

The first vertical tray sidewall section **30** has a beveled corner **31**, Figs. **1** and **5**, and the vertical retainer sidewall sections **48** and **60** have mating beveled corners **61**, Figs. **1** and **3**, thereby to align the retainer with the tray repeatedly in the same orientation.

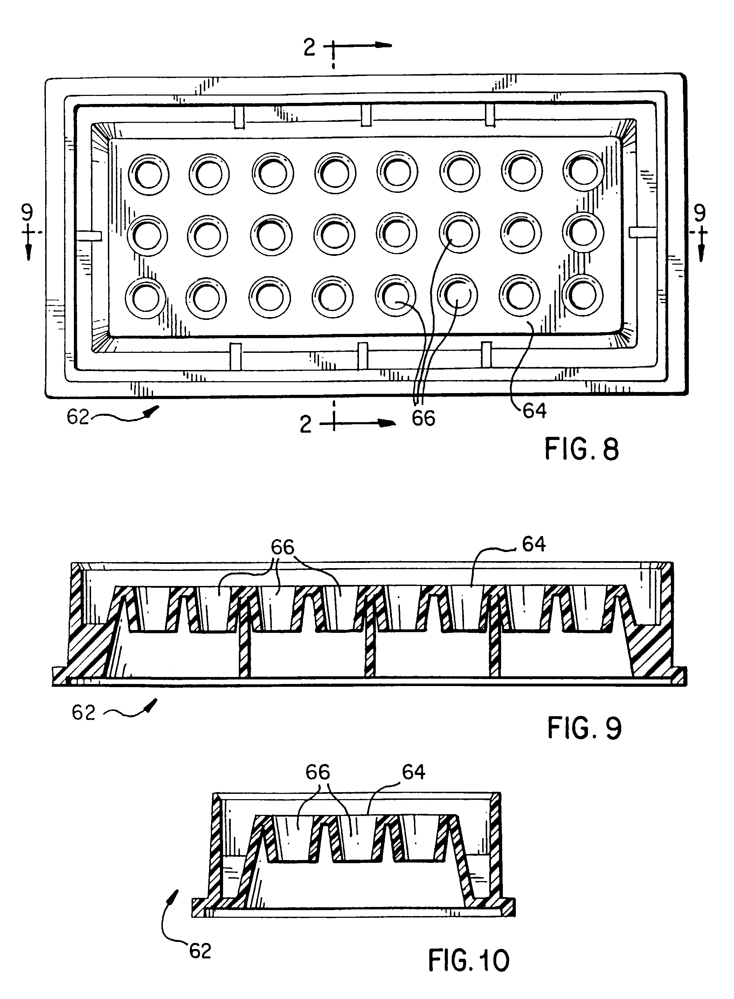

After processing, all of the tubes, such as those indicated at **32**, Fig. **1**, they may be removed simultaneously by lifting the tray **21** out of the PCR instrument. For convenience and storage, the tray **21** with the sample tubes and the retainer **40** in place can be inserted into another plastic component called a base **62**, Fig. **1**. The base **62** is assembled with the tray **21** and the retainer **40** and the sample tubes **32** to form a microtiter plate assembly **68** having a footprint of an industry standard microtiter plate assembly. That is, the base has the outside dimensions and footprint of a standard **24**-well microtiter plate as is shown in Figs. **1**, **8**, **9** and **10**. Fig. **8** is a top plan view of the base **62**, while Fig. **9** is a sectional view taken along the line **9-9** in Fig. **8**. Fig. **10** is a sectional view taken through the base along section line **10-10** in Fig. **8**. The base **62** includes a flat plate section **64** in which an array of twenty four wells **66** with sloped edges is formed. These wells have dimensions and spacing such that when the tray **21** is nested in the base **62**, the holes **44**, **24** and wells **66** are in vertical alignment, and the bottoms of the sample tubes **32** are held in the same relationship to the tray **21** as the sample tubes are held when the frame is mounted in the PCR instrument. The individual sample tubes, though loosely captured between the tray and the retainer, become firmly seated and immobile when the tray is inserted in the base. That is, when the tray **21**, sample tubes **32**, and retainer **40** are seated in the base **62**, the entire assembly becomes the exact functional equivalent of an industry standard **24**-well, for example, microtiter plate, and can be placed in virtually any automatic pipetting or sampling system for a **24**-well industry nicrotiter plates for further processing.

The aforementioned sections of the tray **21** and retainer **40** are preferably molded from reinforced polyester thermoplastic or the equivalent and the sections have a thickness of the order of about 1.27 mm.

It will thus be seen that the present invention does indeed provide a new and improved microtiter plate assembly that is easy to assembly and disassemble and yet gives each sample tube sufficient freedom of motion in all necessary directions to compensate for differing rates of thermal expansion and yet retains them in an array that is compatible with industry standard microtiter plate format.

Although certain particular embodiments of the invention are herein disclosed for purposes of explanation, the invention is not limited by said embodiments. Reference should accordingly be had to the appended claims in determining the scope of the invention.

## Claims

1. A two piece plastic holder assembly (10) for loosely holding a plurality of microtiter sample tubes (12) comprising
a tray (21) having a first plurality of holes (24) for receiving the sample tubes (12),
said tray having opposite vertical end walls (35),
each of said tray end walls having two spaced vertically extending slots (34) and a horizontally extending recess (36) between the slots,
a retainer (40) releasably nestable in said tray (21) having a second plurality of holes (44) corresponding to the plurality of holes (24) in said tray (21) for receiving the sample tubes (12),
said retainer (40) having opposite vertical end walls (52) corresponding to the end walls (35) of said tray,
a U-shaped handle (50) extending horizontally outwardly from each of said opposite retainer end walls (52), each of said handles having two legs (54) which slide into said tray slots (34) respectively when said retainer (40) is nested in said tray (21),
a tab (56) projecting horizontally outwardly from each of the retainer end walls (52) between each of said two legs (54) which snap into said tray end wall recesses (36) respectively when said retainer is nested in said tray,
said retainer (40) having an elongated slot (58) parallel and directly adjacent each of said end walls (52), whereby inwardly directed finger pressure on said U-shaped handle (50) inwardly flexes said opposite ends of said retainer to release said tabs (56) from said tray recesses (36) respectively to facilitate removal of said retainer (40) from said tray (21).

2. Apparatus according to claim 1 further comprising a plastic base (62) having a plurality of wells (66) in a rectangular array compatible with said plurality of holes in said retainer and in said tray,
said wells being dimensioned to snugly accept lower sections of said tubes (12),
said base (62) being nestable with said tray (21), said retainer (40) and said sample tubes (12) to form a microtiter plate assembly (68) having a footprint of an industry standard microtiter plate assembly.

3. Apparatus according to claim 1 wherein said holder assembly (10) is molded from reinforced polyester thermal plastic

4. Apparatus according to claim 1 wherein the wall thickness of said holder assembly (10) is of the order of about 1.27 mm.

5. Apparatus according to claim 1 wherein said tray (21) and said retainer (40) have mating beveled corners (31, 61), thereby to repeatedly align said retainer with said tray in the same orientation.

6. Apparatus according to any of the preceding claims wherein the tray comprises
i) a flat, horizontal rectangular tray plate section (22) containing the first plurality of holes (24),
ii) a first vertical tray sidewall section (30) around said plate (22) extending upwardly,
the rectangular retainer (40) being releasably nestable in said tray (21) over any sample tubes (12) resting in said tray (21), the said retainer comprises
i) a flat, horizontal retainer plate section (42) containing the second plurality of holes (44) in a rectangular array compatible with said first plurality of holes, and
ii) a first vertical retainer sidewall section (48) around said plate (42) extending upwardly.

7. Apparatus according to any of the claims 1 to 6 wherein said tray (21) includes a second vertical tray sidewall section (38) around said tray plate section (22) extending downward from said plate (22).

8. Apparatus according to claim 6 or 7 wherein said retainer (40) includes a second vertical retainer sidewall section (48) around said retainer plate section (42) extending downwardly from said plate (42).

9. Apparatus according to any of the claims 1 to 8 wherein said sample tubes (12) have a cylindrically shaped upper section (14) open at its top end (16) and a closed, tapered lower section (18) extending downwardly therefrom, each tube being of circular cross section and having a circumferential shoulder (20) extending outwardly from said upper section (14) at a position on said upper section spaced from the open end thereof,
said holes (24) in said tray (21) being slightly larger than the outside diameter of the upper section (14) of said tubes but smaller than the outside diameter of said shoulder (20), said first vertical tray sidewall section (30) having a height greater than the height of a tube (12) resting in one of said holes (24), said second vertical tray sidewall section (38) extending downwardly approximately to the bottom of the upper section (14) of a tube (12) resting in one of said holes (24);
said holes (44) in said retainer (40) being slightly larger than the outside diameter of the upper section (14) of said tubes (12) but smaller than the outside diameter of said shoulder (20), and
wherein when said retainer (40) is nested in said tray, the retainer plate section (42) lies slightly above the shoulder (14) of a tube resting in said tray and the first tray sidewall section (30) is about as high as said retainer sidewall section (48), whereby tubes resting in said tray are retained loosely both vertically and laterally.

10. Apparatus according to claim 9 wherein the holes (24) in the tray plate section (22) and in the retainer plate section (42) are larger in diameter than said tubes (12) by about 0.7 mm.

11. Apparatus according to claim 9 or 10 wherein the holes (24) in said tray sections (22) are counter sunk and wherein the underside of the shoulders (14) of said tubes (12) are correspondingly tapered.

12. Apparatus according to any of the claims 1 to 11 wherein said first plurality and said second plurality of holes comprises up to twenty-four holes for receiving up to twenty-four microtiter sample tubes in said holder.

13. Apparatus according to any of the claims 1 to 12 wherein said tray (21) further comprises a plurality of support ribs (28) extending along the underside of said tray plate section between rows of holes, and wherein said retainer (40) further comprises a plurality of support ribs (46) extending along the upperside of said retainer plate section (42) between rows of holes.

## Patentansprüche

1. Zweiteilige Kunststoffbehälterbaugruppe (10) zur losen Aufnahme einer Vielzahl von Mikrotiter-Probenröhrchen (12), die umfasst:
eine Schale (21) mit einer ersten Vielzahl von Löchern (24) zur Aufnahme der Probenröhrchen (12),
wobei die Schale einander gegenüberliegende vertikale Abschlusswände (35) hat,
jede der Schalenabschlusswände zwei beabstandete, sich vertikal erstreckende Einkerbungen (34) und eine sich horizontal erstreckende Aussparung (36) zwischen den Einkerbungen aufweist,
einen Träger (40), der lösbar in die Schale (21) eingesetzt werden kann und eine zweite Vielzahl von Löchern (44) aufweist, die der Vielzahl von Löchern (24) in der Schale (21) entspricht, um die Probenröhrchen (12) aufzunehmen,
wobei der Träger (40) einander gegenüberliegende vertikale Abschlusswände (52) hat, die den Abschlusswänden (35) der Schale entsprechen,
einen U-förmigen Griff (50), der sich von jeder der einander gegenüberliegenden Trägerabschlusswände (52) horizontal nach Außen erstreckt, wobei jeder der Griffe zwei Schenkel (54) hat, jeweils in die Schaleneinkerbungen (34), gleiten, wenn der Träger (40) in der Schale (21) sitzt,
eine Zunge (56), die von jeder der Trägerabschlusswände (52) zwischen jedem der beiden Schenkel (54) nach außen vorsteht und jeweils in die Schalenabschlusswandaussparungen (36) einschnappt, wenn der Träger in der Schale sitzt,
wobei der Träger (40) einen Längsschlitz (58) parallel zu jeder der Abschlusswände (52) und direkt daran angrenzend aufweist, so dass durch nach innen gerichteten Fingerdruck auf den U-förmigen Griff (50) einander gegenüberliegende Ecken des Trägers nach innen gebogen werden und die Zungen (56) jeweils aus den Schalenaussparungen (36) lösen, um so das Entnehmen des Trägers (40) aus dem Träger (21) zu ermöglichen.

2. Vorrichtung nach Anspruch 1, die des weiteren einen Kunststoff-Untersatz (62) mit einer Vielzahl von Vertiefungen (66) in einer rechteckigen Anordnung umfasst, die mit der Vielzahl von Löchern in dem Träger und in der Schale kompatibel ist,
wobei die Vertiefungen so bemessen sind, dass sie die unteren Abschnitte der Röhrchen (12) enganliegend aufnehmen,
der Untersatz (62), die Schale (21), der Träger (40) und die Probenröhrchen (12) ineinander geschachtelt werden können, so dass eine Mikrotiterplattenbaugruppe (68) entsteht, die die gleiche Grundfläche hat wie eine Industriestandard-Mikrotiterplattenbaugruppe.

3. Vorrichtung nach Anspruch 1, wobei die Behälterbaugruppe (10) aus verstärktem Polyester-Thermoplast geformt wird.

4. Vorrichtung nach Anspruch 1, wobei die Wanddicke der Behälterbaugruppe (10) in der Grössenordnung von ungefähr 1,27 mm liegt.

5. Vorrichtung nach Anspruch 1, wobei die Schale (21) komplementäre abgeschrägte Ecken (31, 61) hat, so dass der Träger immer wieder in der gleichen Ausrichtung auf die Schale ausgerichtet werden kann.

6. Vorrichtung nach einem der vorangehenden Ansprüche, wobei die Schale umfasst:
(a) einen flachen, horizontalen, rechteckigen Schalenplattenabschnitt (22), der die erste Vielzahl von Löchern (24) enthält,
(b) einen ersten vertikalen Schalenseitenwandabschnitt (30), um die Platte (22) herum, der sich nach oben erstreckt,
wobei der rechteckige Träger (40) lösbar in die Schale (21) über alle in der Schale (21) sitzenden Probenröhrchen (12) gesetzt werden kann und der Träger umfasst:
(a) einen flachen, horizontalen Trägerplattenabschnitt (42), der die zweite Vielzahl von Löchern (44) in einer rechteckigen Anordnung enthält, die kompatibel zu der ersten Vielzahl von Löchern ist, und
(b) einen ersten vertikalen Trägerseitenwandabschnitt (48), um die Platte (42) herum, der sich nach oben erstreckt.

7. Vorrichtung nach einem der Ansprüche 1 bis 6, wobei die Schale (21) einen zweiten vertikalen Schalenseitenwandabschnitt (38) um den Schalenplattenabschnitt (22) herum enthält, der sich von der Platte (22) aus nach unten erstreckt.

8. Vorrichtung nach Anspruch 6 oder 7, wobei der Träger (40) einen zweiten vertikalen Trägerseitenwandabschnitt (48), um den Trägerplattenabschnitt (42) herum, enthält, der sich von der Platte (42) aus nach unten erstreckt.

9. Vorrichtung nach einem der Ansprüche 1 bis 8, wobei die Probenröhrchen (12) einen zylindrisch geformten oberen Abschnitt (14) aufweisen, der an seinem oberen Ende (16) offen ist, sowie einen geschlossenen, sich verjüngenden unteren Abschnitt (18), der sich von ihm aus nach unten erstreckt, und wobei jedes Röhrchen einen kreisförmigen Querschnitt hat und einen Umfangsabsatz (20) aufweist, der sich von dem oberen Abschnitt (14) an einer Position an dem oberen Abschnitt, die von dem offenen Ende desselben beabstandet ist, nach außen erstreckt,
wobei die Löcher (24) in der Schale (21) etwas größer sind als der Außendurchmesser des oberen Abschnitts (14) der Röhrchen, jedoch kleiner als der Außendurchmesser des Absatzes (20), und wobei die Höhe des ersten vertikalen Schalenseitenwandabschnitts (30) größer ist als die Höhe eines Röhrchens (12), das in einem der Löcher (24) sitzt, und der zweite Schalenseitenwandabschnitt (38) sich ungefähr bis zum unteren Ende des oberen Abschnitts (14) eines Röhrchens (12), das in einem der Löcher (24) sitzt, nach unten erstreckt;
wobei die Löcher (44) in dem Träger (40) etwas größer sind als der Außendurchmesser des oberen Abschnitts (14) der Röhrchen, jedoch kleiner als der Außendurchmesser des Absatzes (20), und
wobei, wenn der Träger (40) in der Schale sitzt, der Trägerplattenabschnitt (42) geringfügig über dem Absatz (20) eines Röhrchens, das in dem Träger sitzt, liegt, und der erste Trägerseitenwandabschnitt (30) ungefähr genauso hoch ist wie der Trägerseitenwandabschnitt (48), so dass Röhrchen, die in dem Träger sitzen, sowohl vertikal als auch in Querrichtung lose gehalten werden.

10. Vorrichtung nach Anspruch 9, wobei der Durchmesser der Löcher (24) in dem Schalenplattenabschnitt (22) und in dem Trägerplattenabschnitt (42) um ungefähr 0,7 mm größer ist als der der Röhrchen (12).

11. Vorrichtung nach Anspruch 9 oder 10, wobei die Löcher (24) in den Schalen abschnitten (22) eingesenkt sind, und wobei die Unterseiten der Absätze (20) der Röhrchen (12) sich entsprechend verjüngen.

12. Vorrichtung nach einem der Ansprüche 1 bis 11, wobei die erste Vielzahl und die zweite Vielzahl von Löchern bis zu 24 Löchern zum Aufnehmen von bis zu 24 Mikrotiter-Probenröhrchen in dem Behälter umfassen.

13. Vorrichtung nach einem der Ansprüche 1 bis 12, wobei die Schale (21) des weiteren eine Vielzahl von Stützstegen (28) umfasst, die sich an der Unterseite des Schalenplattenabschnitts zwischen Reihen von Löchern erstrecken, und wobei der Träger (40) des weiteren eine Vielzahl von Stützstegen (46) umfasst, die sich an der Oberseite des Trägerplattenabschnitts (42) zwischen Reihen von Löchern erstrecken.

## Revendications

1. Un ensemble support en matière plastique (10) en deux pièces, pour maintenir de façon lâche une pluralité de tubes échantillons de microtitrage (12); comprenant :
un plateau (21) comportant une première pluralité de trous (24) pour recevoir les tubes d'échantillon (12),
ledit plateau ayant des parois d'extrémité verticales (35) opposées,
chacune desdites parois d'extrémité de plateau ayant deux fentes (34) espacées s'étendant verticalement et une cavité (36) s'étendant horizontalement entre les fentes,
un élément de retenue (40) pouvant être imbriqué de façon désolidarisable dans ledit plateau (21) ayant une deuxième pluralité de trous (44) correspondant à la pluralité de trous (24) ménagés dans ledit plateau (21) afin de recevoir les tubes échantillons (12),
ledit élément de retenue (40) ayant des parois d'extrémité (52) verticales opposées correspondant aux parois d'extrémité (35) dudit plateau,
une poignée en forme de U (50) s'étend horizontalement vers l'extérieur depuis chacune desdites parois d'extrémité (52) opposées d'éléments de retenue, chacune desdites poignées ayant deux pieds (54) qui coulissent dans lesdites fentes de plateau (34), respectivement, lorsque ledit élément de retenue (40) est imbriqué dans ledit plateau (21),
une patte (56) faisant saillie horizontalement vers l'extérieur depuis chacune desdites parois d'extrémité (52) d'éléments de retenue, entre chacun desdits deux pieds (54) qui s'encliquètent dans lesdites cavités de paroi d'extrémité de plateau (36), respectivement, lorsque ledit élément de retenue est imbriqué dans ledit plateau,
ledit élément de retenue (40) ayant une fente (58) allongée parallèle et directement adjacente à chacune desdites parois d'extrémité (52), de manière que la pression des doigts orientés vers l'intérieur, exercée sur ladite poignée en forme de U (50), fasse fléchir à l'intérieur lesdites extrémités opposées dudit élément de retenue pour libérer lesdites pattes (56) desdites cavités de plateau (36), respectivement, afin de faciliter l'enlèvement dudit élément de retenue (40) vis-à-vis dudit plateau (21).

2. Dispositif selon la revendication 1, comprenant en outre une base en matière plastique (62) dotée d'une pluralité de puits (66) placés en une matrice à côtés rectangulaires compatible avec ladite pluralité de trous ménagés dans ledit élément de retenue et dans ledit plateau,
lesdits puits étant dimensionnés pour recevoir de façon ajustée des sections inférieures desdits tubes (12),
ladite base (62) étant imbricable avec ledit plateau (21), ledit élément de retenue avec ledit plateau (21), ledit élément de retenue (40) et lesdits tubes échantillon (12) pour former un ensemble de plaques de microtitrage (68) ayant une empreinte d'un ensemble de plaques de microtitrage d'un standard industriel.

3. Dispositif selon la revendication 1, dans lequel ledit ensemble support (10) est moulé à partir d'une matière thermoplastique, telle que du polyester renforcé.

4. Dispositif selon la revendication 1, dans lequel l'épaisseur de paroi dudit ensemble support (10) est de l'ordre d'environ 1,27 mm.

5. Dispositif selon la revendication 1, dans lequel ledit plateau (21) et ledit élément de retenue (40) ont des angles (31, 61) chanfreinés, adaptés, de manière à aligner répétitivement ledit élément de retenue avec ledit plateau dans la même orientation.

6. Dispositif selon l'une des revendications précédentes, dans lequel le plateau comprend :
i) une section de plaque de plateau (22) rectangulaire, horizontale, plate contenant la première pluralité de trous (24),
ii) une première section de paroi latérale de plateau (30) verticale autour de ladite plaque (22) s'étendant vers le haut,
l'élément de retenue rectangulaire (40) étant imbricable de façon désolidarisable dans ledit plateau (21) sur l'un quelconque desdits tubes échantillons (12) placés dans ledit plateau (21), ledit élément de retenue comprenant :
i) une section de plaque d'élément de retenue (42) horizontale, plate, contenant la deuxième pluralité de trous (44) en une matrice rectangulaire compatible avec ladite première pluralité des trous,
ii) une première section de paroi latérale d'élément de retenue (48) verticale autour de ladite plaque (42) et s'étendant vers le haut.

7. Dispositif selon l'une quelconque des revendications 1 à 6, dans lequel ledit plateau (21) comprend une deuxième section de paroi de plateau (38) verticale autour de ladite section de plaque de plateau (22), s'étendant vers le bas, vis-à-vis de ladite plaque (22).

8. Dispositif selon l'une des revendications 6 ou 7, dans lequel ledit élément de retenue (40) comprend une deuxième section de paroi latérale d'élément de retenue (48) verticale autour de ladite section de plaque d'élément de retenue (42), s'étendant vers le bas depuis ladite plaque (22).

9. Dispositif selon l'une quelconque des revendications 1 à 8, dans lequel lesdits tubes échantillons (12) ont une section supérieure (14) à forme cylindrique, ouverte à son extrémité supérieure (16), et une section inférieure (18) effilée, fermée, s'étendant vers le bas à partir de celle-ci, chaque tube ayant une section transversale circulaire et ayant un épaulement circonférentiel (20) s'étendant vers l'extérieur depuis ladite section supérieure (14), en une position sur ladite section supérieure, espacée de son extrémité ouverte,
lesdits trous (24) ménagés dans ledit plateau (21) étant légèrement plus grands que le diamètre extérieur de la section supérieure (14) desdits tubes, mais plus petit que le diamètre extérieur dudit épaulement (20), ladite première section de paroi latérale (30) de plateau verticale ayant une hauteur supérieure à la hauteur d'un tube (12) placé dans l'un desdits trous (24), ladite deuxième section de paroi latérale de plateau (38) verticale s'étendant vers le bas, à peu près jusqu'au fond de la section supérieure (14) d'un tube (12) placé dans l'un desdits trous (24),
lesdits trous (44) ménagés dans ledit élément de retenue (40) étant légèrement plus grands que le diamètre extérieur de ladite section supérieure (14) desdits tubes (12), mais inférieurs au diamètre extérieur dudit épaulement (20), et
dans lequel, lorsque ledit élément de retenue (40) est imbriqué dans ledit plateau, la section de plaque d'élément de retenue (42) est placée légèrement au-dessus de l'épaulement (14) d'un tube placé dans ledit plateau et la première section de paroi latérale de plateau (30) est à peu près aussi haute que ladite de paroi latérale d'élément de retenue (48), de manière que les tubes placés dans ledit plateau soient retenus de façon lâche à la fois verticalement et latéralement.

10. Dispositif selon la revendication 9, dans lequel les trous (24), ménagés dans la section de plaque de plateau (22) et dans la section de plaque d'élément de retenue (42), sont de diamètre supérieur à celui desdits tubes (12), la différence étant d'environ 0,7 mm.

11. Dispositif selon la revendication 9 ou 10, dans lequel les trous (24) ménagés dans lesdites sections de plateau (22) sont dotés d'une contre-dépouille et dans lequel la face inférieure des épaulements (14) desdits tubes (12) est dotée d'un effilement correspondant.

12. Dispositif selon l'une quelconque des revendications 1 à 11, dans lequel ladite première pluralité et ladite deuxième pluralité de trous sont constituées de vingt-quatre trous devant recevoir jusqu'à vingt-quatre tubes d'échantillonnage de microtitrage dans ledit support.

13. Dispositif selon l'une quelconque des revendications 1 à 12, dans lequel ledit plateau (21) comprend en outre une pluralité de nervures support (28) s'étendant sur la face inférieure de ladite section de plaque de plateau entre des rangées de trous, et dans lequel ledit élément de retenue (40) comprend en outre une pluralité de nervures support (48) s'étendant sur la face inférieure de ladite section de plaque d'élément de retenue (42) entre des rangées de trous.
